(19) Europäisches Patentamt

European Patent Office

Office européen des brevets

(11) **EP 1 037 158 A2**

(12) **EUROPEAN PATENT APPLICATION**

(43) Date of publication:
20.09.2000 Bulletin 2000/38

(51) Int. Cl.$^7$: **G06F 19/00**

(21) Application number: 00302025.2

(22) Date of filing: 14.03.2000

(84) Designated Contracting States:
**AT BE CH CY DE DK ES FI FR GB GR IE IT LI LU MC NL PT SE**
Designated Extension States:
**AL LT LV MK RO SI**

(30) Priority: **15.03.1999 US 124453 P**

(71) Applicant:
**WHITEHEAD INSTITUTE FOR BIOMEDICAL RESEARCH
Cambridge, MA 02142 (US)**

(72) Inventors:
• **Tamayo, Pablo
Cambridge, Massachusetts 02139 (US)**
• **Mesirov, Jill
Belmont, Massachusetts 02478 (US)**
• **Lander, Eric S.
Cambridge, Massachusetts 02139 (US)**
• **Golub, Todd R.
Newton, Massachusetts 02164 (US)**

(74) Representative:
**Holdcroft, James Gerald, Dr. et al
Graham Watt & Co.,
Riverhead
Sevenoaks, Kent TN13 2BN (GB)**

(54) **Methods and apparatus for analyzing gene expression data**

(57)    The present invention relates to methods and apparatus for grouping or clustering gene expression patterns from a plurality of genes. The invention utilizes a Self Organizing Map to cluster the gene expression patterns into groups that exhibit similar patterns. The clustering enables one to easily analyze gene expression data from potentially thousands of genes.

Fig. 1

**Description**

BACKGROUND OF THE INVENTION

The expression of genes is studied to provide insight into gene function and discover new methods of treatment for a variety of genetically related diseases. However, the ability does not yet exist to analyze the expression of multiple genes simultaneously, especially when genes that are being expressed are subject to several variables, conditions and/or parameters. Scientists have long since struggled to analyze such massive datasets of gene expression.

Accordingly, a need exists for methods and/or apparatus for analyzing large sets of gene expression patterns. In particular, a need exists to identify groups of genes that express similar patterns under particular conditions. Such information would be extremely useful as an analytical tool in developing or identifying drug targets and therapies.

SUMMARY OF THE INVENTION

The invention relates to methods and apparatus for analyzing, clustering, or grouping gene expression data. In particular, the invention relates to a method for clustering or grouping a plurality of datapoints, wherein each datapoint is a series of gene expression values. The gene expression values are obtained from a gene (e.g., in a cell) that is subjected to at least one condition. A dataset is a series of gene expression values obtained across multiple genes subjected to a condition. Gene expression products (mRNA, proteins) are obtained from cells which have been subjected to at least one condition, such as time; exposure to changes in temperature, pH, or other growth/incubation conditions; exposure to an agent, such as a drug or drug candidate, or toxin. The method comprises receiving the gene expression values of the datapoints and, using a self organizing map (SOM), clustering the datapoints such that the datapoints that exhibit similar patterns are clustered together into respective clusters. The method then involves providing an output that indicates the clusters of the datapoints. The method may also include filtering out any datapoints that exhibit insignificant change (e.g., little or no change) in the gene expression values, such that working datapoints remain. The method optionally may also include normalizing the gene expression value of the working datapoints. The self organizing map is formed of a plurality of Nodes, N, and clusters the datapoints according to a competitive learning routine, for example, $f_{i+1}(N) = f_i(N) + \tau(d(N,N_P), i) (P - f_i(N))$ , wherein i = number of iterations, N= the node of the self organizing map, $\tau$ = learning rate, P = the subject working datapoint, d = distance, $N_p$ = node that is mapped nearest to P, and $f_i(N)$ is the position of N at i. The method may optionally include rescaling the gene expression values to account for variations.

The invention also pertains to methods for assessing expression patterns of two or more genes in a cell, wherein the expression patterns are represented by a plurality of datapoints, and each datapoint is a series of gene expression values for a gene. The method comprises receiving the gene expression values of the datapoints and, using a self organizing map, clustering the datapoints such that the datapoints that exhibit similar patterns are clustered together into respective clusters. The method also comprises providing an output indicating the clusters of the datapoints, and analyzing the output to determine the similarities or differences between the expression patterns of the genes. The method can also comprise filtering out any datapoints that exhibit insignificant changes in the gene expression, and/or normalizing the gene expression value of the working datapoints. Particularly, the self organizing map is formed of a plurality of Nodes, N, and clusters datapoints according to the competitive learning routine stated above.

The steps described above and herein can be used for a variety of applications involving gene expression analyses. The applications are numerous and are described herein in detail. Accordingly, the invention relates to methods of characterizing expression patterns of a plurality of genes present in a sample having unknown characteristics. For example, a sample to be assessed for gene expression is obtained from an individual and subjected to a multiplicity of diagnostic tests. The gene expression patterns for the diagnostic tests are represented by a plurality of datapoints. Each datapoint is a series of gene expression values corresponding to the result of a diagnostic test. The method comprises receiving the gene expression values of the datapoints from the diagnostic tests, and, using a self organizing map, clustering the datapoints such that datapoints that exhibit similar patterns are clustered together into respective clusters. The method also comprises providing the output indicating the clusters of the datapoints, and comparing the output of the gene expression patterns of the unknown sample against a control to thereby characterize gene expression patterns of the sample. These steps allow one to determine characteristics of the sample, or to classify the sample. The sample from the individual can be cells, lysed cells, cellular material suitable for determining gene expression, or other material (e.g., lymph, urine, sputum, supernatant, etc.) containing gene expression products.

The present invention also relates to methods for identifying a drug target by assessing the expression patterns of two or more genes from cells. The cells, referred to as test cells or test sample, are subjected to an agent or condition. The expression patterns are represented by a plurality of datapoints, and each datapoint is a series of gene expression values for a gene. The method comprises receiving the expression values of the datapoints, clustering the datapoints with a self organizing map and comparing the clusters from the genes exposed to the agent or condition, to a control (e.g., clusters produced by using the same method of gene expression patterns for cells of the same type as the test cells treated in the same manner, except that they have not been exposed to the agent or condition). The method also comprises providing an output that indicates a drug target. The comparing step can be performed by a person or by a computer system.

The invention also relates to computer apparatus for clustering or grouping a plurality of datapoints, wherein each datapoint is a series of gene expression values for a gene. The apparatus comprises a source (e.g., input device) of gene expression values of the datapoints, a processor routine that is responsive to the input device and utilizes a self organizing map for clustering datapoints from the source. The datapoints that exhibit similar patterns are clustered together into respective clusters. The apparatus further comprises an output device, coupled to the processor routine, that indicates the clusters of the datapoints. The computer apparatus may also comprise a filter coupled to the source, for filtering out any datapoints that exhibit an insignificant change in gene expression value, such that working datapoints remain. The apparatus can also comprise a normalizing process, that is coupled to the filter, for normalizing the gene expression value of the working datapoints. The self organizing map is formed of a plurality of Nodes, N, and clusters of datapoints according to a competitive learning routine, for example, $f_{i+1}(N) = f_i(N) + \tau(d(N,N_P), i) (P - f_i(N))$, wherein i = number of iterations, N = the node of the self organizing map, $\tau$ = learning rate, P = the subject working datapoint, d = distance, $N_p$ = node that is mapped nearest to P, and $f_i(N)$ is the position of N at i. The apparatus may also include an output device that displays at least one representative datapoint from each cluster.

The present invention's methods and apparatus allow one to interpret the expression pattern of thousands of genes quickly and easily, thereby revolutionizing molecular biology and the study of genes. The invention allows for the extraction of fundamental patterns of gene expression and can be used to organize thousands of genes into biologically relevant groups. Such information provides new insight about gene function and its involvement in various pathways, as well as targets for new drugs for the treatment of diseases, such as cancer or genetic diseases or disorders.

BRIEF DESCRIPTION OF THE DRAWINGS

Figure 1 is a schematic illustrating the principle behind the Self-Organizing Maps (SOM). Initial geometry of nodes in 3x2 rectangular grid is indicated by solid lines connecting the nodes. Datapoints are represented by black dots, six nodes of SOM by large circles, and trajectories by arrows.

Figure 2 is a block diagram of a network employing SOMs of the present invention.

Figures 3A-D1 are graphical representations of a SOM utilizing a 6x5 grid of the yeast cell cycle.

Figure 3E1 is a graph showing the gene expression pattern of Cluster 29 in detail.

Figure 3F1 is a three dimensional graph showing the centroids for SOM-derived clusters 29, 14, 1, and 5, corresponding to G1, S, G2 and M phases of cell cycle.

Figure 3G1 is a three dimensional graph showing the centroids for groups of genes identified by visual inspection as having peak expression in G1, S, G2 or M phases of the cell cycle.

Figures 4A-L are graphic representations showing the gene expression for HL-60 cells treated with TPA for 0, 0.5, 4 or 24 hours. The expression levels of more than 6000 genes were measured at each time point. The 567 genes passing the variation filter were grouped by a 4x3 SOM.

Figures 5A-X are graphic representations showing the gene expression during Hematopoietic Differentiation. The 1036 genes varying in at least one of four cell lines were used to generate a 6x4 SOM. Time courses for four cell lines are shown, separated by blank space. Order of cell lines is: HL-60+TPA, U937+TPA, NB4+ATRA, Jurkat+TPA.

Figures 6A-B summarize the experiments performed under various conditions for a Yeast Cell Cycle analysis. This summary and all data obtained for the experiments can be found at http://genome-www.stanford.edu/cellcycle.

DETAILED DESCRIPTION OF THE INVENTION

The invention relates to methods and apparatus for clustering (e.g., grouping) gene expression patterns from a plurality of genes. New technologies (e.g., array technologies) provide the ability to analyze gene expression for thousands of genes. These new technologies have made it straight forward to monitor simultaneously the expression patterns of thousands of genes. Richer experimental designs involving hundreds of samples and conditions are able to be easily analyzed using the present invention. Until now, comparison of gene expression was impossible or has been a painstakingly slow process. Prior to the invention, analysis of hundreds or thousands of genes was very time consuming. The invention significantly speeds up the process of analyzing gene expression patterns by grouping or clustering genes that have similar expression patterns and extracting fundamental patterns of gene expression from data.

A common computational approach is hierarchical clustering. Datapoints are forced into a strict hierarchy of nested subsets so that the closest pair of points is grouped and replaced by a single point representing their set average, and the next closest pair of points is treated similarly, and so on. The datapoints are thus fashioned into a phylogenetic tree, whose branch lengths represent the degree of similarity between the sets.

Hierarchical clustering, however, has a number of shortcomings for the study of gene expression. Strict phylogenetic trees are best suited to situations of true hierarchical descent, such as in the evolution, of species and are not designed to reflect the multiple distinct ways in which expression patterns can be similar. This problem is exacerbated as the size and complexity of the dataset grows. Hierarchical clustering suffers from lack of robustness, non-uniqueness and inversion problems that complicate interpretation of the hierarchy. Finally, the deterministic nature of hierarchical clustering can cause points to be grouped based on local decisions, with no opportunity to re-evaluate the clustering. It is known that the resulting trees can lock in accidental features, reflecting idiosyncrasies of the agglomeration rule.

Applicants have discovered that Self Organizing Maps (SOMs) have a number of features that make them particularly well suited to clustering and analysis of gene expression patterns. In contrast to the rigid structure of hierarchical clustering, the strong priors of Bayesian clustering, and the non-structure of k-means clustering they are ideally suited to exploratory data analysis. SOMs allow one to impose partial structure on the clusters and facilitate easy visualization and interpretation. They have good computational properties, because they are easy to implement, are reasonably fast, and are scalable to large datasets.

Applications of the invention include, for example, assessing the function of unknown genes, assessing the function of genes in cells that undergo certain metabolic processes or stages (e.g., cell cycle or cell death), assessing the function of genes that are subject to particular conditions, or identifying genes that are a drug target. The present methods and apparatus can be used to assess the applicability of a particular treatment for an individual who has a certain gene expression profile, or the likelihood an individual has or will have a genetic disease. These applications are described herein in greater detail. The invention also includes any and all applications for which gene expression is currently being used, and/or will be used in the future. As described herein, the present invention is applicable to (can cluster) gene expression data regardless of the means by which it is obtained.

The invention clusters or groups gene expression data. A cluster is a group of gene expression patterns that are similar. The gene expression patterns for each gene are represented by a datapoint. A datapoint refers to a series of (more than one) gene expression values. The gene expression values, as described herein, can be obtained across various samples, trials, experiments, or conditions. A dataset is a series of values of gene expression across multiple genes (e.g., corresponding to one condition, experiment, sample, or trial). In some applications, for example, when clustering gene expressions of a sample having unknown characteristics and comparing the clusters to a control, the datapoint is a series of gene expression values within the sample, condition, experiment, or trial (e.g., when analyzing unknown properties of a sample), rather than across them. Those particular applications in which the definition of the datapoint varies are described herein, and/or are readily apparent in light of the application of the invention.

The methods and/or apparatus for clustering or grouping gene expression data involves analyzing data obtained from a variety (more than one) of possible conditions. Different cell types can also be analyzed for different gene expression values. A snap shot of gene expression values is taken during the experiment. The cells which express the genes can be subjected to a variety of conditions, such as time, pressure, exposure to changes in temperature, pH, or other growth/incubation conditions; light or sound waves; cell stages or metabolic processes; exposure to various compounds or agents (e.g., drugs, drug candidate or toxin), alone or in combination. The compounds or agents can inhibit or enhance gene expression. For example, one can subject the cells/sample to the compound to determine the effect on gene expression, or one can subject the cells to allow certain metabolic or cell cycle processes to occur and measure the gene expression at various stages. A wide variety of conditions can be studied, so long as those conditions are suitable for gene expression. Conditions suitable for gene expression are those which are now used for measuring gene expression, or will be used in the future.

Gene expression products are proteins or nucleic acids that are involved in transcription or translation (e.g., mRNA, tRNA, rRNA, or cRNA). The present invention can effectively be used to analyze proteins or nucleic acids that are involved in transcription or translation. The nucleic acid levels measured can be derived directly from the gene or, alternatively, from a corresponding regulatory gene. All forms of products can be measured including spliced variants. Similarly, gene expression can be measured by assessing the level of protein or derivative thereof translated from mRNA. Sources of gene expression products are cells, lysed cells, cellular material for determining gene expression, or material containing gene expression products (e.g.,lymph, urine, sputum, supernatant, etc.).

The gene expression value measured is the actual numeric value obtained from an apparatus that can measure such levels. The values can be raw values from the apparatus. Such data is obtained, for example, from a gene chip probe array (Affymetrix, Inc.)(U.S. Patent Nos. 5,631,734, 5,874,219, 5,861,242, 5,858,659, 5,856,174, 5,843,655, 5,837,832, 5,834,758, 5,770,722, 5,770,456, 5,733,729, 5,556,752, all which are incorporated herein by reference in their entirety). The gene chip contains a variety of probe arrays that adhere to the chip in a predefined position. The chip contains thousands of probes. Nucleic acids (e.g., mRNA) from an experiment or sample which has been subjected to particular conditions hybridizes to the probes which exist on the chip. The nucleic acid to be analyzed (e.g.,

the target) is isolated, amplified and labeled with a detectable label, (e.g., $^{32}$P or fluorescent label), prior to hybridization to the gene chip probe arrays. Once hybridization occurs, the arrays are inserted into a scanner which can detect patterns of hybridization. The hybridization data are collected as light is emitted from the labeled groups, which is now bound to the probe array. The probes that perfectly match the target produce a stronger signal than those that have mismatches. Since the sequence and position of each probe on the array are known, by complementarity, the identity of the target nucleic acid applied to the probe is determined. The amount of light detected by the scanner becomes raw data that the invention applies and utilizes. The gene chip probe array is only one example of obtaining the raw gene expression value. Other methods for obtaining gene expression values are well known in the art.

The gene expression values are preferably rescaled to account for variables across experiments or conditions. Such variables depend on the experimental design the researcher chooses. See Examples 6 and 7. The preparation of the data preferably also involves filtering and/or normalizing the values prior to subjecting the gene expression values to clustering. The data, throughout its preparation and processing, may appear in table form. Partial tables appear throughout and are meant to illustrate principals and concepts of the invention. For example, Table 1 is a partial gene expression table.

TABLE 1

| This is an example of a gene/experiment expression table: | | | | | |
| --- | --- | --- | --- | --- | --- |
| gene\experiment | Exp. 1 | Exp. 2 | Exp. 3 | Exp. 4 | Exp. 5, etc. |
| gene 1 | 5 | 50 | 500 | 450 | 200 |
| gene 2 | 200 | 800 | 3300 | 500 | 500 |
| gene 3 | 30 | 31 | 29 | 30 | 31 |
| gene 4 | 5000 | 4000 | 3000 | 2000 | 1000 |
| gene 5, etc. | 10 | 30 | 50 | 70 | 90 |

Filtering the gene expression values involves eliminating any datapoint in which the gene expression value exhibits no change or an insignificant change, e.g., across experiments or conditions. Once the genes are filtered out then the subset of gene expression datapoints that remain are referred to herein "working datapoints.". The purpose of filtering out these values is to avoid skewing the gene expression clustering. Basically, the filtering out of gene expression values are those which exhibit a flat expression pattern over the experiments or conditions. Although these datapoints (e.g., gene expression patterns) are eliminated, they can still have biological significance or importance. For example, to learn that a genes expression remains unaffected by a compound provides important information about the gene, and its non-susceptibility to the compound. Hence, in addition to providing an output of clustered gene expression data, the invention can also provide a list of those genes whose expression level exhibited an insignificant change, with or without the particular expression level. Table 2 contains the working datapoints from Table 1 (e.g., the gene expression values from Table 1 with those genes exhibiting an insignificant change in the gene expression pattern being eliminated).

TABLE 2

| This is an example of a gene/experiment expression table: | | | | | |
| --- | --- | --- | --- | --- | --- |
| gene\experiment | Exp. 1 | Exp. 2 | Exp. 3 | Exp. 4 | Exp. 5, etc. |
| gene 1 | 5 | 50 | 500 | 450 | 200 |
| gene 2 | 200 | 800 | 3300 | 500 | 500 |
| gene 4 | 5000 | 4000 | 3000 | 2000 | 1000 |
| gene 5, etc. | 10 | 30 | 50 | 70 | 90 |

The present invention also preferably involves normalizing the levels of gene expression values. The absolute level of the gene expression is not as important as the shape of the gene expression (e.g., whether the expression level rises or falls). Normalization allows for the clustering or comparing of gene expression values whose level could be a thousand times the absolute value of expression level for another gene. Preferably, normalization occurs using the

following equation:

$$NV = (\frac{GEV - AGEV}{SDV}),$$

wherein NV is the normalized value, GEV is the gene expression value, AGEV is the average gene expression value, and SDV is the standard deviation of the gene expression value. The normalization occurs, for example, across experiments, samples, or conditions. Table 3, below, is the partial data table containing gene expression values which have been normalized, utilizing the values in Table 2.

TABLE 3

| This is an example of a gene/experiment expression table: | | | | | |
|---|---|---|---|---|---|
| gene\experiment | Exp. 1 | Exp. 2 | Exp. 3 | Exp. 4 | Exp. 5, etc. |
| gene 1 | -1.043441147 | -0.844479911 | 1.145132445 | 0.924064405 | -0.181275792 |
| gene 2 | -0.677144363 | -0.204718063 | 1.763724853 | -0.440931213 | -0.440931213 |
| gene 4 | 1.264911064 | 0.632455532 | 0 | -0.632455532 | -1.264911064 |
| gene 5, etc. | -1.264911064 | -0.632455532 | 0 | 0.632455532 | 1.264911064 |

Once the gene expression values are prepared, then the data is clustered or grouped. The invention utilizes SOMs for clustering or grouping expression patterns. SOM is a competitive learning routine.

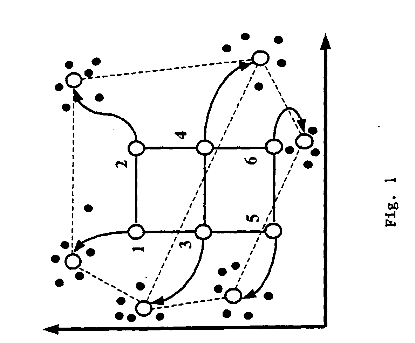

SOMs are constructed by first choosing a geometry of 'nodes'. Preferably a 2 dimensional grid (e.g., a 3x2 grid) is used, but other geometries can be used, as described herein. The nodes are mapped into k-dimensional space, initially at random and then interactively adjusted. Figure 1 illustrates Nodes 1,2,3,4,5, and 6 in such a grid in space. Each iteration involves randomly selecting a datapoint P and moving the nodes in the direction of P. The closest node $N_P$ is moved the most, while other nodes are moved by smaller amounts depending on their distance from $N_P$ in the initial geometry. In this fashion, neighboring points in the initial geometry tend to be mapped to nearby points in k-dimensional space. The process continues for several (e.g., 20,000-50,000) iterations.

SOMs impose structure on the data, with neighboring nodes tending to define 'related' clusters. An SOM based on a rectangular grid is analogous to an entomologist's specimen drawer, with adjacent compartments holding similar insects. Alternative structures can be imposed on the data through different initial geometries, such as grids, rings and lines with different numbers of nodes.

The number of nodes in the SOM can vary according to the data. For example, the user can increase the number of Nodes to obtain more clusters. The proper number of clusters allows for a better and more distinct representation of the particular gene pattern of the cluster. The grid size corresponds to the number of nodes. For example a 3x2 grid contains 6 nodes and a 4x5 grid contains 20 nodes. As the SOM algorithm is applied to the gene expression data, the nodes move toward the gene cluster over several iterations. The number of Nodes directly relates to the number of clusters. Therefore, an increase in the number of Nodes results in an increase in the number of clusters. Having too few nodes tends to produce patterns that are not distinct. Additional clusters result in distinct, tight clusters of expression. The addition of even more clusters beyond this point does not result any fundamentally new patterns. For example, one can choose a 3x2 grid, a 4x5 grid, and/or a 6x7 grid, and study the output to determine the most suitable grid size.

A variety of SOM algorithms exist that can cluster gene expression datapoints. The invention utilizes any SOM routine (e.g., or competitive learning routine that clusters the expression patterns), and preferably, uses the following SOM routine.

$$f_{i+1}(N) = f_i(N) + \tau(d(N,N_P), i) (P - f_i(N)),$$

wherein i = number of iterations, N= the node of the self organizing map, $\tau$ = learning rate, P = the subject working datapoint, d = distance, $N_p$ = node that is mapped nearest to P, and $f_i(N)$ is the position of N at i.

After the expression patterns are clustered or grouped, the output is provided (e.g., to a printer, display or to another software package such as graphic software for display). One can then analyze the genes in the cluster. The analysis depends on the experimental design and can include ascertaining the affect of the conditions or agent, the relatedness of one gene to others, or determining the similarities and/or differences among the genes.

The analysis often depends on comparing the clusters to a control. A control is gene expression data from cells that can provide a baseline or standard against which to measure. The control differs depending on the experimental design. Expression values of a control is obtained from cells that, for example, have not been exposed to the conditions being analyzed. The control is a used to measure the unknown variable. A control is a comparison group or standard that differs from the condition being studied. The control can be a negative or positive control. The term is known in the art.

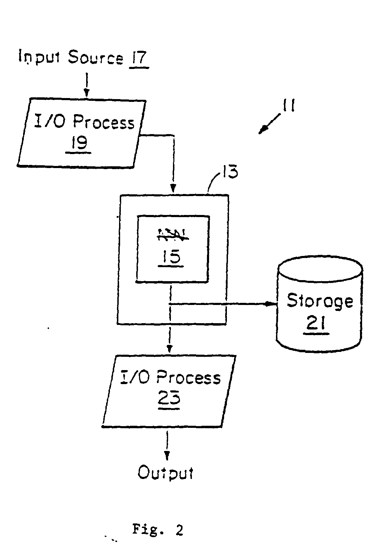

Referring to Figure 2, a computer system embodying a software program 15 (e.g., a processor routine) of the present invention is generally shown at 11. The computer system 11 employs a host processor 13 in which the operation of software programs 15 are executed. An input device or source such as on-line data from a work-station terminal, a sensor system, stored data from memory and the like provides input to the computer system 11 at 17. The input is pre-processed by I/O processing 19 which queues and/or formats the input data as needed. The pre-processed input data is then transmitted to host processor 13 which processes the data through software 15. In particular, software 15 maps the input data to an output pattern and generates clusters indicated on output for either memory storage 21 or display through an I/O device, e.g., a work-station display monitor, a printer, and the like. I/O processing (e.g., formatting) of the content is provided at 23 using techniques common in the art. The computer system according to the invention is useful in applications including, but not limited to, gene expression recognition, drug target predictions, and gene/cell segmentation analysis.

Receiving the gene expression data refers to delivering data, which may or may not be pre-processed (e.g., rescaled, filtered, and/or normalized), to the software 15 (e.g., processing routine) that clusters the gene expression patterns. A processor routine refers to a set of commands that carry out a specified function. The invention utilizes a processor routine in which a SOM algorithm clusters gene expression patterns. Once the software 15 clusters the datapoints, then an output is provided which indicates the clusters. Providing an output refers to providing the datapoints to an output (I/O) device.

The invention has numerous applications. As described herein and in the Examples, the present invention can be used for analyzing genes whose function is unknown, or at least unknown in the conditions tested in the experimental design. The conditions can be any condition already utilized to assess gene expression or a condition utilized in the future. Such conditions include time, temperature, cell stages, pressure, light waves (e.g., ultra violet waves, infrared waves ) sound waves or a compound. The compound can be one that inhibits or enhances gene expression. The invention an also be used to analyze different cell types having different gene expression values.

When time is a condition, one can analyze processes of the cell, such as cell cycle. Example 1, 2 and 4 illustrate this application of the present invention. Samples of mRNA were taken from yeast cells at various stages of the cell cycle. The amount of time that was necessary for the cell to progress to the particular stages passed and mRNA samples were taken. The invention is not limited to cell cycle, but virtually any metabolic, biochemical, or replicative process that a cell can undergo. Basically, the gene expression product is obtained from the stages being measured, using known methods and quantified. The gene expression product, preferably mRNA, is labeled (e.g., $^{32}$P) and allowed to hybridize (e.g., bind to nucleic acid complement) with known and pre-defined nucleic acid, oligonucleotide probes. The amount of hybridized nucleic acid is measured, and values are determined. These gene expression values are preferably preprocessed and then clustered according to the present invention, as described herein.

The invention also allows one to analyze and identify regulatory genes or genes that are co-regulated (e.g., genes that are involved in similar pathways). For example, genes that have similar expression or are expressed under the same condition likely act together or are involved in similar processes. Hence, the present invention can be used to determine genes that are expressed or are important for regulating a particular pathway. Genes involved in the pathway are targets for drugs or therapy.

Another application of the invention is identifying a drug target. A drug target refers to a compound, gene or nucleic acid or fragment thereof, protein or protein fragment that is a candidate for treatment of a disease. A disease is one that changes or has an effect on gene expression. Such diseases include diseases having gene defects or alterations, infections caused by virus, cancers, diseases caused by toxins, disorders involving trauma to cells, and genetically related diseases (e.g., a set of genes in which at least one has a defect in its expression and causes the disease or particular phenotype related to the disease). The cell or cellular material that is capable of expressing genes are subjected to the compound or a compound combination to be tested. Cells that have been exposed to the compound to be tested as well as cells that have not been exposed (e.g., a control) can be assessed. Other controls include cells being exposed to certain media or conditions, depending on the experimental design. Therefore, one should extract gene expression products from a control as well as the cells being tested with the compound. The levels are measured and clustered or grouped according to the invention. The software clusters both the control gene expression data and gene expression data from the cells being tested with the compound (e.g., the test sample). The invention includes comparing the gene expression clusters from the control to the test sample. This step can be performed by a person or apparatus and can be performed before or after the output is provided. For example, a gene that exhibits change in gene expression due to the compound's presence will not appear in the same cluster, as compared to the control in which the cells were not exposed to this compound. Multiple genes can be affected by the compound to be tested. One can readily focus on the genes that are affected by the compound (or those not affected, depending on the experimental design). Prior to this invention, one would need to compare thousands of genes manually which takes an inordinate amount of time. In seconds, utilizing the invention provides this information to analyze or assess a drug target. Any cellular system can be studied so long as gene expression products can be obtained. The invention also includes the drugs targeted from the methods described herein.

Yet another application of the present invention is analysis of samples from an individual (e.g., a diagnostic application). A gene profile can be obtained utilizing the methods and apparatus of the invention. For example, persons who have a disease also have a particular gene expression profile. The invention implicates any disease, as defined herein. A sample from persons having the disease has certain gene expression clustering when the sample is exposed to particular conditions (e.g., diagnostic tests), as described herein. A control, standard or baseline can be a gene profile from a person or group of persons with the disease (positive control) and/or a profile from a person or group of persons without the disease (negative control). An individual whose sample is to be tested is obtained. The sample can be subjected to the same conditions as the control. A person having the disease will exhibit similar gene expression clustering as the positive control and dissimilar gene expression clustering as the negative control. Additionally, the application of the invention can determine the probability or likelihood that the individual being tested will contract the disease. For example, a disease can be the result of numerous gene defects, or gene defects that are subjected to certain environmental affects. Hence, the application can convey the number of genes and the significance of their expression, in comparison to the control.

The invention can also be utilized to determine characteristics or properties of a sample (e.g., a sample having unknown characteristics). For example, the invention can be used to ascertain whether a sample is susceptible or likely to benefit from a particular treatment. One can obtain a tissue sample from any part of the body, for example, the colon, breast, kidney and lungs. To ascertain whether any of these samples would benefit from a particular treatment (e.g., cancer treatment), the invention is applied by obtaining gene expression products from the cells of the various tissue samples under particular conditions (e.g., diagnostic tests). A control can be samples which are known to be successful when subjected to treatment (positive control), and/or known not to be successful when subjected to treatment (negative control). The samples and control samples are subjected to diagnostic tests that indicate that the characteristic (e.g., susceptibility to cancer treatment). The gene expression products are quantified and the gene expression values are pre-processed. The values are pre-processed, as described herein, except they are, preferably, not filtered, but they are normalized. The datapoint, in this particular application, is represented by a series of gene expression values across genes and within the diagnostic test, to enable one to compare the patterns of diagnostic tests as established by the gene expression data. Characteristics of the sample to be tested are determined. Conceptually, the table of gene expression values is inverted.

Table 4

| illustrates a partial set of datapoints. | | | | | |
|---|---|---|---|---|---|
| Gene \ Experiment | Colon | Leukemia | Melanoma | Breast | Renal |
| CYC1 Cytochrome c-1 (D00265) | 313 | 597 | 595 | 205 | 283 |
| CYP3A7 Cytochrome P450 IIIA7 (D00408) | -4 | 7 | 3 | 9 | 5 |
| TYMSThymidylate synthase (D00596) | 156 | 431 | 401 | 289 | 222 |
| FECH Ferrochelatase (D00726) | 33 | 24 | 20 | 72 | 26 |
| T-CELL Antigen CD7 (D00749) | 18 | 7 | 14 | 2 | 27 |

The samples being tested that fall into similar clusters as the positive control indicate that the tissue would be successful in the treatment as well. Virtually, any properties or characteristics can be ascertained, depending on the Experimental design.

Yet another embodiment of the invention is its application to screening individuals for determining whether the individual is a candidate for a particular drug or treatment regimen. Prior to this invention, several drugs do not reach the market place because they work in a small percentage of the individuals tested. Clinical studies often reveal that a drug is successful in some individuals, but not successful in others. The genetic variability that exists among a patient population can be the cause of a drug's failure. The present invention can be used to cluster and analyze the gene expression products of an individual, who has undergone successful treatment with the drug, under certain conditions. For example, the drug in question could be platelet inhibitor and the patient population comprises individuals with a history of coronary disease. Suitable conditions, to which samples of the individuals are subjected, can be, for example, conditions that relate to platelet aggregation. A platelet rich sample can be exposed to various platelet aggregation agonists and antagonists as well as the drug. Controls can be clusters of gene expression levels from individuals in which treatment was (positive control) and was not (negative control) successful. After establishing controls, potential candidates (e.g., individuals having a history of coronary disease such as previous angina or myocardial infarctions) for drug can be screened to determine the probability of a successful treatment with the drug. The clusters of gene expression from the individual being screened is compared with the clusters of individuals who have had successful and unsuccessful treatment. Clusters of gene expression similar to an individual who has received successful treatment with the drug indicates that the individual being screened would also be a good candidate for treatment. Gene expression clusters similar to the control of individual who underwent unsuccessful treatment indicates a poor candidate for treatment. The screening process is applicable to all drug screening, and not limited to cardiac drug treatments.

The invention can be applied to numerous applications that involve gene expression. The experimental design and application of the invention depends on the piece of information that is being obtained. The unknown piece of information can be: the unknown function of a gene in known conditions, the effect of unknown conditions to known gene function, or the unknown likelihood of successful treatment by a drug (e.g., for a specific tissue sample). The invention's applications are numerous and are not limited to the examples described herein. The invention applies to virtually any experimental design that involves the expression of numerous genes.

EXEMPLIFICATION

Example 1: Self-Originating Map and Methods Used in Assessing Gene Expression for Yeast Cell Cycle and Hematopoietic Differentiation.

The computer package, GENECLUSTER™, to produce and display SOMs of gene expression data encompasses the invention. The program was then applied to various datasets involving the yeast cell cycle and hematopoietic differentiation, to evaluate its ability to assist in interpretation of gene expression.

Self-Organizing Maps: An SOM has a set of nodes with a simple topology (e.g., two-dimensional grid) and a distance function $d(N_1,N_2)$ on the nodes. Nodes are interactively mapped into k-dimensional 'gene expression' space (in which the i-th coordinate represents the expression level in the i-th sample). The position of node N at iteration i is denoted $f_i(N)$. The initial mapping $f_0$ is random. On subsequent iterations, a datapoint P is selected and the node $N_P$ that maps nearest to P is identified. The mapping of nodes is then adjusted by moving points toward P by the formula:

$$f_{i+1}(N) = f_i(N) + \_\tau(d(N,N_P), i)\,(P - f_i(N)).$$

The 'learning rate' $\tau$ decreases with distance of node N from $N_P$ and with iteration number i. The point P used at each iteration is determined by random ordering of the n datapoints generated once and recycled as needed. The function $\tau$ is defined by $\tau(x,i) = 0.02\,T/(T + 100\,i)$ for $x = \rho(i)$ and $\tau(x,i) = 0$ otherwise, where radius $\rho(i)$ decreases linearly with i ($\rho(0) = 3$) and eventually becomes zero and T is the maximum number of iterations. GENECLUSTER™ is written in C, runs under UNIX and requires a Web browser. It is available from the authors. Figure 1 shows hypothetical trajectories of nodes as they migrate to fit data during successive iterations of the SOM algorithm.

Data pre-processing: A variation filter was used to eliminate genes that did not change significantly across samples. Genes were eliminated if they did not show a relative change of X and an absolute change of Y units, with (X,Y) = (2,35) for yeast data and (X,Y) = (3,100) for human data. Expression levels were then normalized to have mean 0 and variance 1. For yeast data, expression levels were normalized within each of the two cell cycles. For the human data, expression levels were normalized within the time points for each cell line.

Cell Culture: HL-60 and U937 cells were provided by American Type Culture Collection, Jurkat cells by S. Burakoff, and NB4 cells line by M. Lanotte. ATRA-resistant lines are described in the art. Cells were grown in RPMI 1640 with 10% fetal bovine serum. HL-60, U937 and Jurkat cells were stimulated with 10 nM TPA (Sigma) for 0, 0.5, 6 or 24 hours; NB4 cells were stimulated with 1 uM *all-trans* retinoic acid (ATRA; Sigma) for 0, 6, 24, 48 or 72 hours. Final concentration for DMSO stimulations was 1.25%.

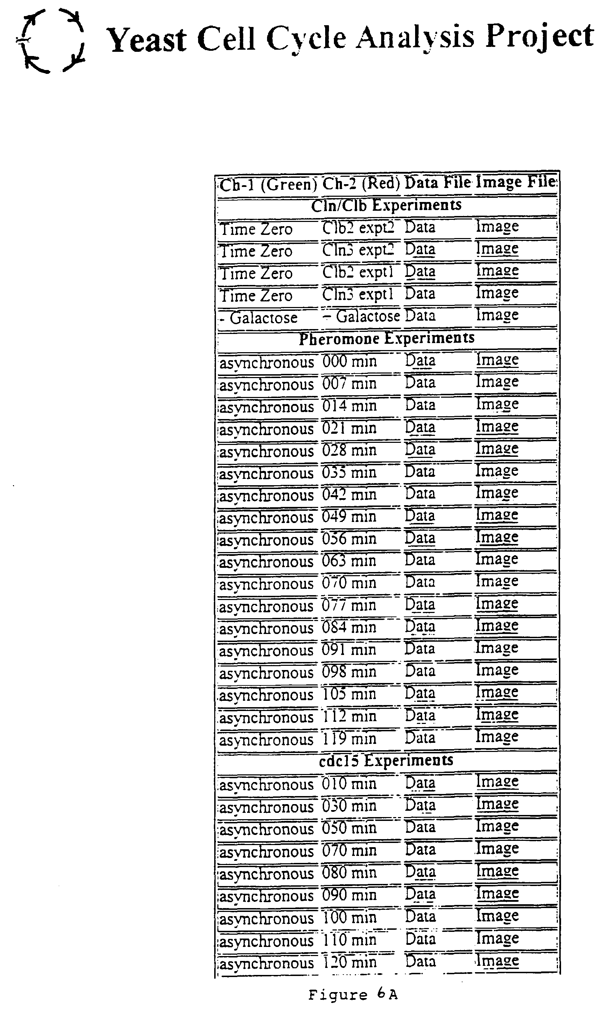

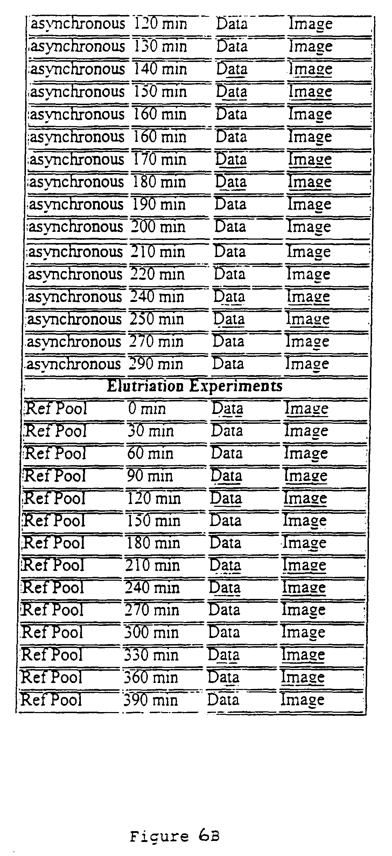

Yeast Experiments: Yeast data was downloaded from http://genome-www.stanford.edu/cellcycle. The 90 minute time point was excluded because of difficulties with scaling. See Figures 6A-B.

Expression Analysis: A detailed protocol is at http://www.genome.wi.mit.edu/MPR, and pertinent portions of it can also be found in Example 5. Briefly, 1 μg mRNA was used to generate first strand cDNA using a T7-linked oligo-dT primer. Following second strand synthesis, *in vitro* transcription (Ambion) was performed with biotinylated UTP and CTP (Enzo), resulting in 40-80 fold linear amplification of RNA. 40 μg of biotinylated RNA was fragmented to 50-150 nucleotide size prior to overnight hybridization to Affymetrix HU6000 arrays. Arrays contain probe sets for 6416 human genes (5223 known genes and 1193 ESTs). Because probe sets for some genes are present more than once on the array, the total number on the array is 7227. Following washing, arrays were stained with streptavidinphycoerythrin (Molecular Probes) and scanned on a Hewlett-Packard scanner. Intensity values were scaled such that overall intensity for each chip of the same type was equivalent. Intensity for each feature of the array was captured using GeneChip software (Affymetrix, Inc.), and a single raw expression level for each gene was derived from the 20 probe pairs representing each gene using a trimmed mean algorithm. A threshold of 20 units was assigned to any gene with a calculated expression level below 20, since discrimination of expression below this level could not be performed with confidence.

Northern Blotting: 10-20 μg of total RNA was electrophoresed through denaturing agarose gels and transferred to Hybond-N nylon membranes (Amersham). Hybridization was performed using Rapid-Hyb buffer (Amersham). A 476 basepair G0S2 probe was generated corresponding to nucleotides 41-516 of the published sequence (GenBank M69199). Probes were $^{32}$P-labelled by random hexamer priming (Stratagene).

Example 2: Results of the Clustering of the Yeast Cell Cycle Gene Expression Patterns.

GENECLUSTER™ accepts an input file of expression levels from any gene profiling method (e.g., oligonucleotide arrays or spotted cDNA arrays), together with a geometry for the nodes.

The program begins with two pre-processing steps that greatly improve the ability to detect meaningful patterns. First, genes are passed through a variation filter to eliminate those with no significant change across the samples. This prevents nodes from being attracted to large sets of invariant genes. Second, the expression level of each gene is normalized across experiments. This focuses attention on the 'shape' of expression patterns rather than on absolute levels of expression.

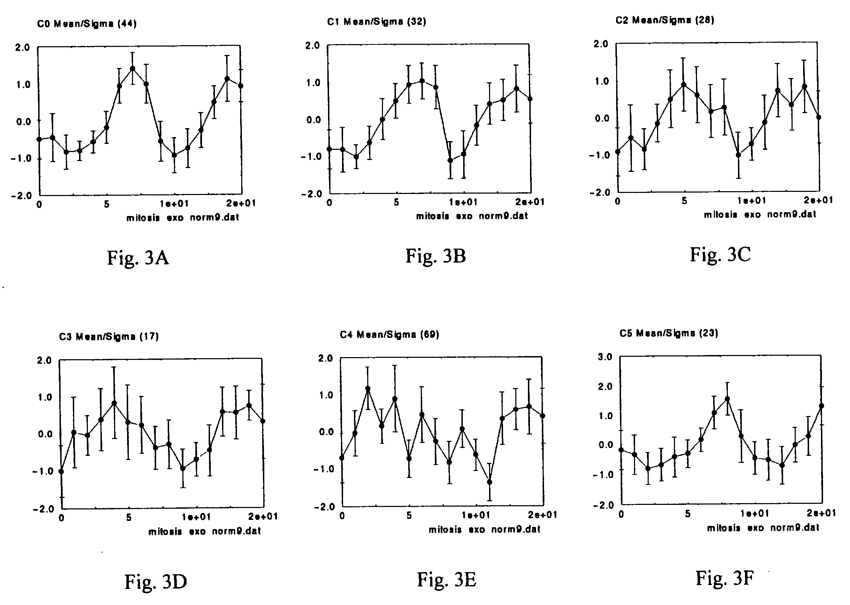

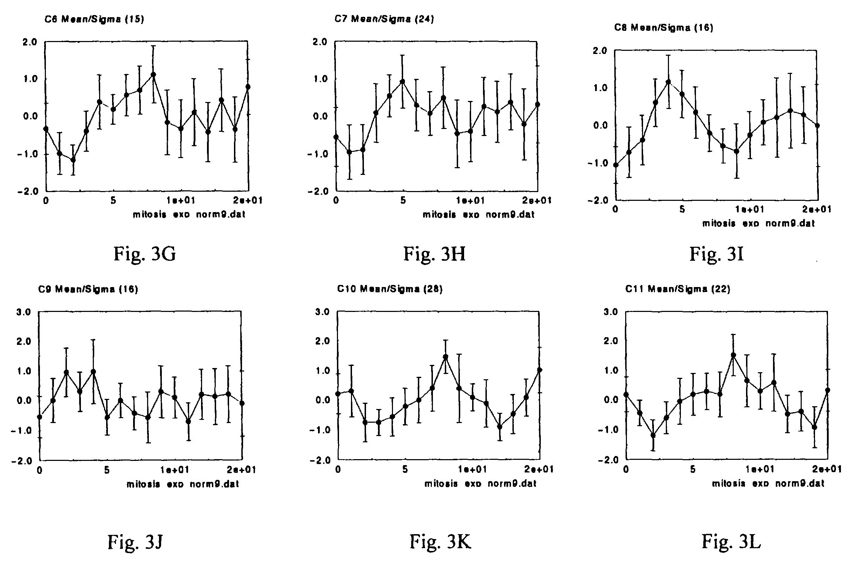

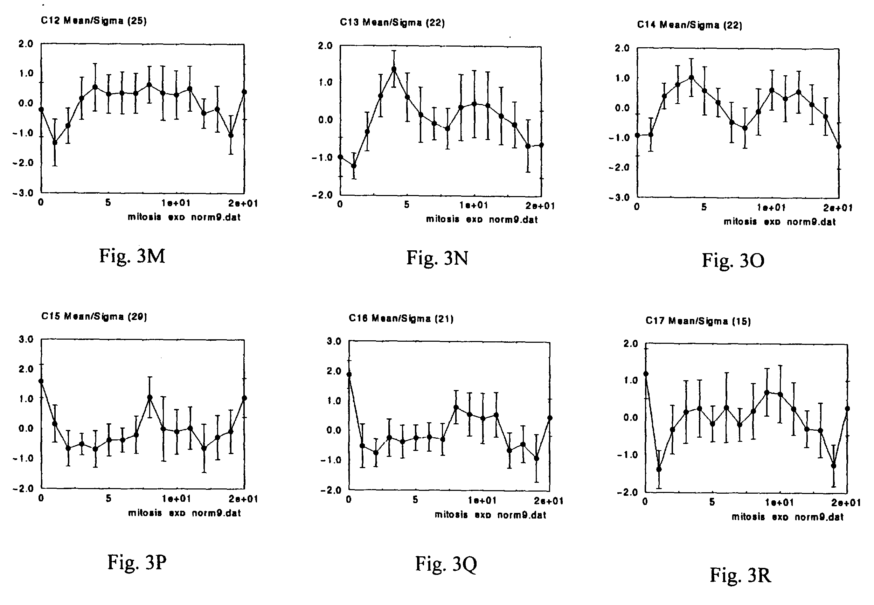

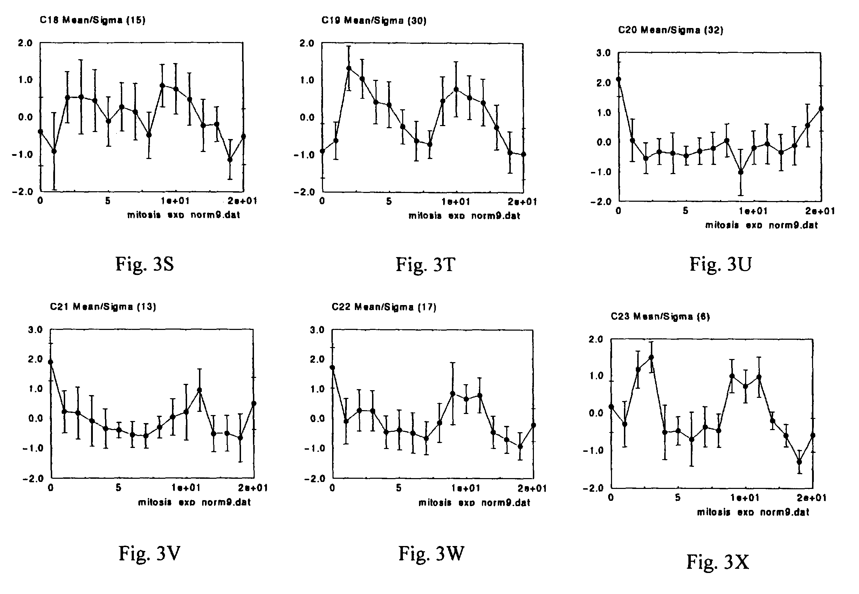

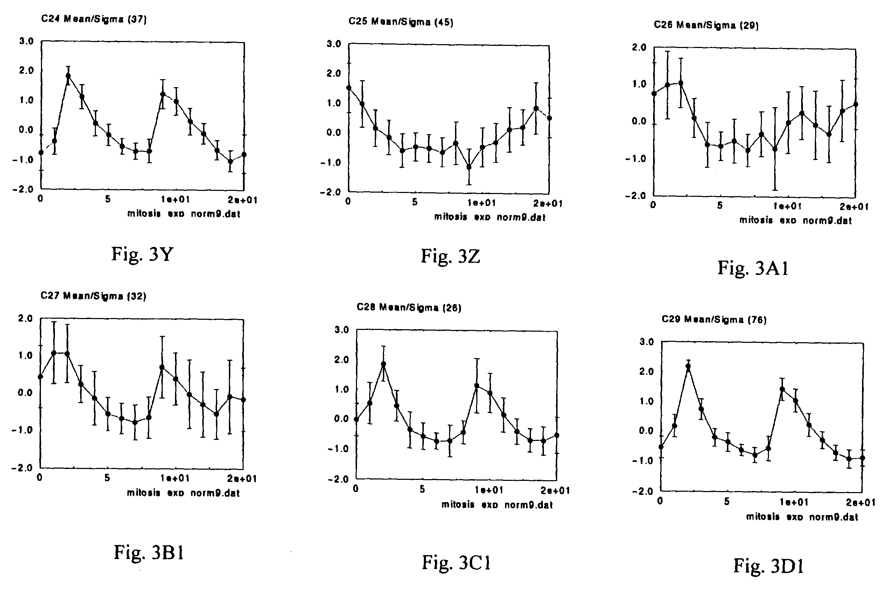

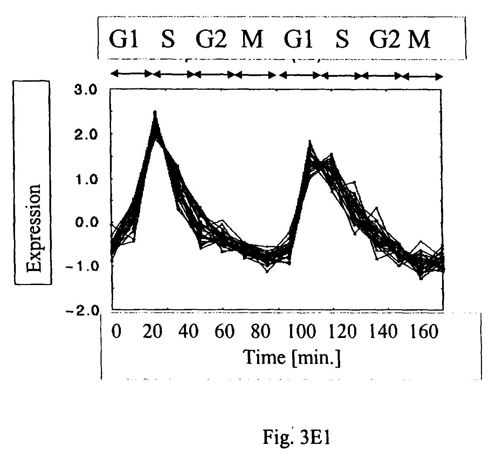

An SOM is then computed, typically in about 1 minute for large datasets, such as below. GENECLUSTER uses a Web-based interface to visualize the clusters. Each cluster is represented by its average expression pattern, making it easy to discern similarities and differences among the patterns. (See Figure 3A-D1) The variation around the pattern can be visualized by means of 'error bars' or by overlaying the patterns of all members of the cluster. (See Figure 3E1)

SOMs are particularly well suited for exploratory data analysis, to expose the fundamental patterns in the data. The underlying structure can be readily explored by varying the geometry of the SOM. With only a few nodes, one tends not to see distinct patterns and there is large within-cluster scatter. As nodes are added, distinctive and tight clusters emerge. Beyond this point, the addition of further nodes tends to produce no fundamentally new patterns. Although there is no strict rule governing such exploratory data analysis, straightforward inspection quickly identified an appropriate SOM geometry in each of the examples below.

Yeast Cell Cycle: GENECLUSTER™ was tested on a published dataset, to determine whether it could automatically expose known patterns without using prior knowledge. For this purpose, data was used from a recent study of Cho, R. *et al* (1998) *Molecular Cell* 2, 65-73. In the study, the researchers synchronized S. *cerevisiae* in G1, released the cells, and collected RNA at 10 min intervals over two cell cycles (160 min). Expression levels of 6,218 yeast ORFs were measured using oligonucleotide arrays. From the set of genes passing a variation filter, the authors used visual inspection to identify 416 genes showing peaks of expression in early G1, late G1, S, G2 or M phase.

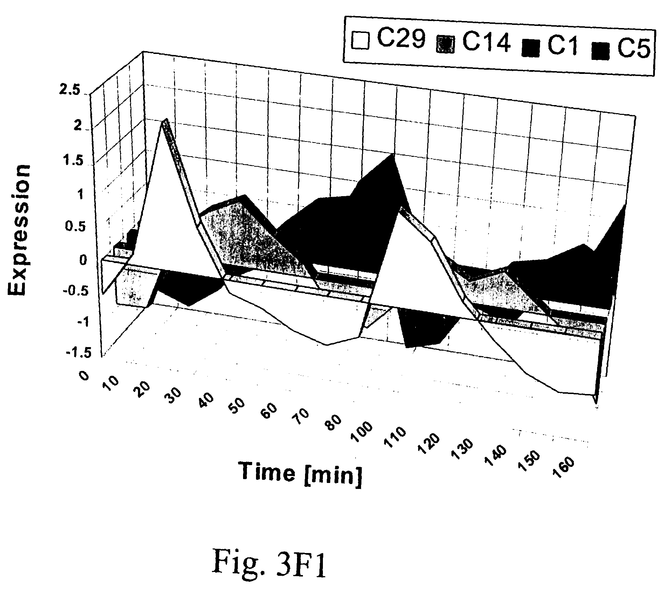

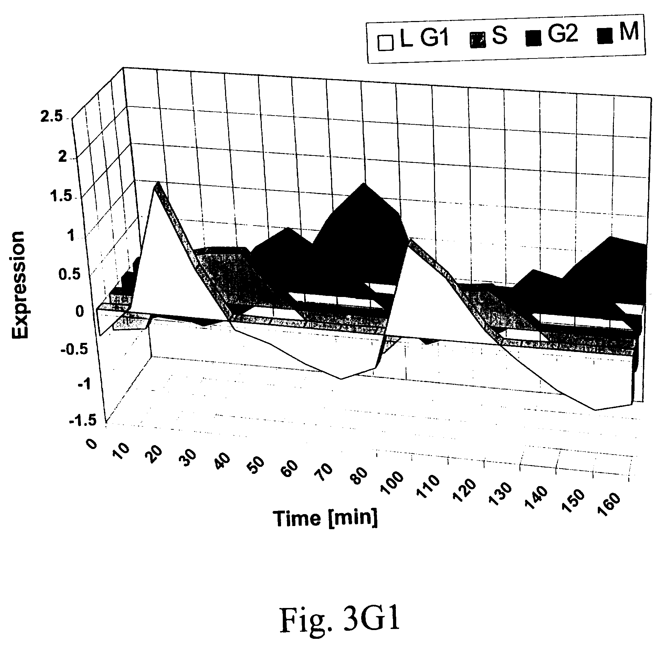

GENECLUSTER™ was used to re-analyze the data, rapidly settling on a 6x5 SOM. As shown in Figure 3A-D1, the SOM automatically and quickly (computation time 82 secs) extracted the cell-cycle periodicity as among the most prominent features in the data. Figure 3A-D1 show 828 genes which were involved in the yeast cell cycle and passed the variation filter. They were grouped into 30 clusters. Each cluster is represented the centroid (average or representative pattern) for genes in the cluster. Expression level of each gene was normalized to have mean 0 and standard deviation 1 across time points. Expression levels are shown on y-axis and time points on x-axis. Error bars indicate standard deviation of average expression. *n* indicates number of genes within each cluster. Note that multiple clusters exhibit periodic behavior, and that adjacent clusters have similar behavior. The neighboring Clusters 24, 28 and 29, for example, contain genes with peak expression in late G1 phase (25-45 min and 85-105 min; See Figures 3A-3D1). Figure 3E1 shows Cluster 29 which contains 76 genes exhibiting periodic behavior with peak expression in late G1. Normalized expression pattern of 30 genes nearest the centroid are shown. The genes agree well with those identified by visual inspection. Of the 105 late G1-peaking genes that passed our variation filter, 91 (87%) were contained in the three G1-associated clusters identified by the SOM. Of the 14 remaining genes, 7 were located in neighboring clusters. More broadly, the SOM-derived clusters corresponding to the G1, S, G2 and M phases of the cell cycle (Figure 3F1) closely match those identified visually by Cho *et al.*, (Figure 3G1).

Example 3: Results of the Clustering of the Hematopoietic Differentiation Gene Expression Pattern.

The present invention was used to analyze human hematopoietic differentiation. This process is largely controlled at the transcriptional level, and blocks in the developmental program likely underlie the pathogenesis of leukemia. Cell lines modeling the differentiation process have been extensively used over the past decade to study expression of dozens of individual genes. Our goal was to take a more global approach by creating a reference database describing the behavior of some 6000 genes.

The myeloid leukemia cell line HL-60, which undergoes macrophage differentiation upon treatment with the phorbol ester TPA was studied. Nearly 100% of HL-60 cells become adherent and exit the cell cycle within 24 hours of TPA treatment. To monitor this process at the transcriptional level, anti-sense cRNA was prepared from cells harvested at 0, 0.5, 4 and 24 hrs after TPA stimulation (see Example 1). Samples were then hybridized to expression-monitoring arrays from Affymetrix, Inc., containing oligonucleotide probes for 5223 known human genes and 1193 expressed sequence tags (ESTs), and hybridization intensities were determined for each gene. The list of genes on the arrays and all expression data are available at

http://www.genome.wi.mit.edu/MPR.

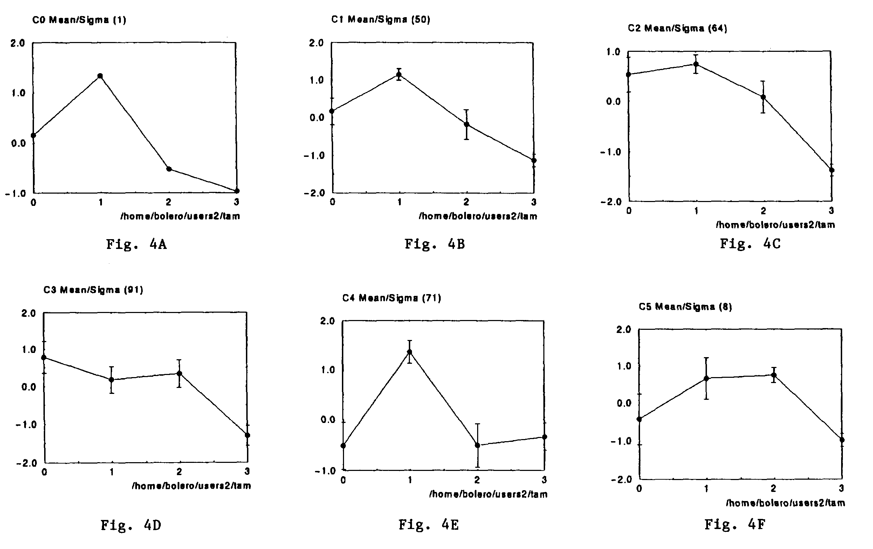

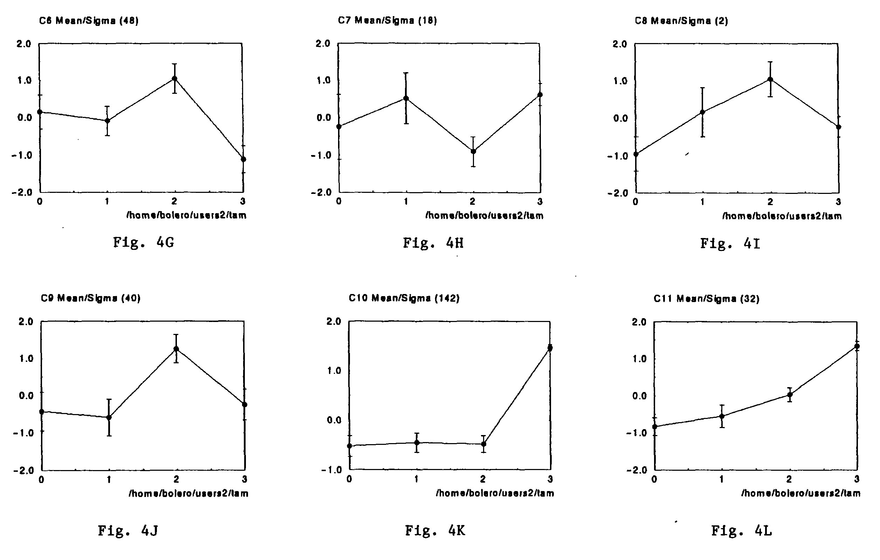

567 genes (9%) passed the variation filter, exhibiting significant change across the four time points, and their expression levels were normalized. A 4x3 SOM was used to organize the genes into twelve clusters. (See Figures 4A-L) Although generated without preconceptions, the clusters correspond to patterns of clear biological relevance. Most of the known genes found to be regulated have, in fact, been previously identified in the extensive literature on macrophage differentiation. Our study, however, identified the vast majority of these genes in a single experiment and also uncovered additional ones not previously known to be regulated.

Cluster 11, for example, contains 32 genes with gradual induction over the time course, during which time cells gradually lose proliferative capacity and acquire hallmarks of the macrophage lineage. Four of the genes are duplicates on the array, reducing the cluster to 28 distinct genes (Table 4). Two are ESTs for which no coding sequence is available. The remaining 26 can be divided into 18 that would be expected based on current knowledge of hematopoietic differentiation (such as the anti-apoptosis genes Bfl-1 and A20, and Macrophage Inflammatory Protein $1\alpha$ (MIP1$\alpha$)) and 8 that seem unexpected.

Table 4

| Genes in Cluster 11 (TPA-induced genes in HL-60 cells) | |
|---|---|
| Expected: | Unexpected: |
| Macrophage Inflammatory Protein 1 alpha | GLVR1 Leukemia virus receptor 1 |
| BFL-1 (Bcl-2 related) | PTPN12 Protein tyrosine phosphatase, non-receptor type 12 |
| PEA-15 Major astrocytic phosphoprotein | FKBP25 FK506-binding protein |
| CD83 antigen | CSNK1A1 Casein kinase 1, alpha 1 |
| DTR Diphtheria toxin receptor (heparin-binding EGF-like growth factor) | CSNK2A2 Casein kinase 2, alpha prime polypeptide |
| JUNB proto-oncogene | RPL3 Ribosomal protein L3 |
| P4HA Procollagen-proline, 2-oxoglutarate 4-dioxygenase (proline 4-hydroxylase), alpha polypeptide | RPL4 Ribosomal protein L4 |
| DAF Decay accelerating factor for complement (CD55) | HIP, putative tumor suppressor (HNC6) |
| EGR2 Early growth response 2 | EST, GenBank accession # H80240 |
| SLP-76 76 kDa tyrosine phosphoprotein | EST, GenBank accession #T53118 |
| TNFAIP1 Tumor necrosis factor alpha inducible protein A20 | |
| KNG Kininogen | |
| Fc-epsilon-receptor gamma-chain | |
| | Tryptophanyl-tRNA synthetase |
| BTG1 B-cell translocation gene 1 | |
| RASA1 GTPase-activating protein ras p21 (RASA) | |
| CRFB4 Cytokine receptor family II, member 4 | |

Table 4 (continued)

| Genes in Cluster 11 (TPA-induced genes in HL-60 cells) | |
| --- | --- |
| Expected: | Unexpected: |
| Homeo box c1 protein | |

Four of the unexpected genes (FKBP25, caseine kinases I and II, and HIP) suggest that an immunophilin-mediated pathway plays a role in macrophage differentiation. FKBP25 is a member of the immunophilin family of FK506-binding proteins which play important roles in protein folding and trafficking. Caseine kinase II is involved in the activation of another immunophilin FKBP52. The HIP protein interacts with the molecular chaperone protein hsc70, which in turn acts in concert with immunophilins and anti-apoptotic proteins.

Cluster 10 has 142 genes showing late induction. These include many genes known to be involved in macrophage differentiation (e.g. CSF1 receptor, IL1β and Cathepsin B). Cluster 2 contains 64 genes showing down-regulation upon terminal differentiation induced by TPA. These include cell-cycle-related genes, such as those encoding cyclin D2, cyclin D3, CDK2 and PCNA. Cluster 4 has 71 genes whose expression peaks within 30 min of TPA treatment, suggesting an immediate early response. These include serum response factor (SRF) and the early growth response gene EGR1.

These results suggest that the SOM captured the predominant patterns of gene regulation in this simple model of macrophage differentiation.

Hematopoietic Differentiation across four cell lines:

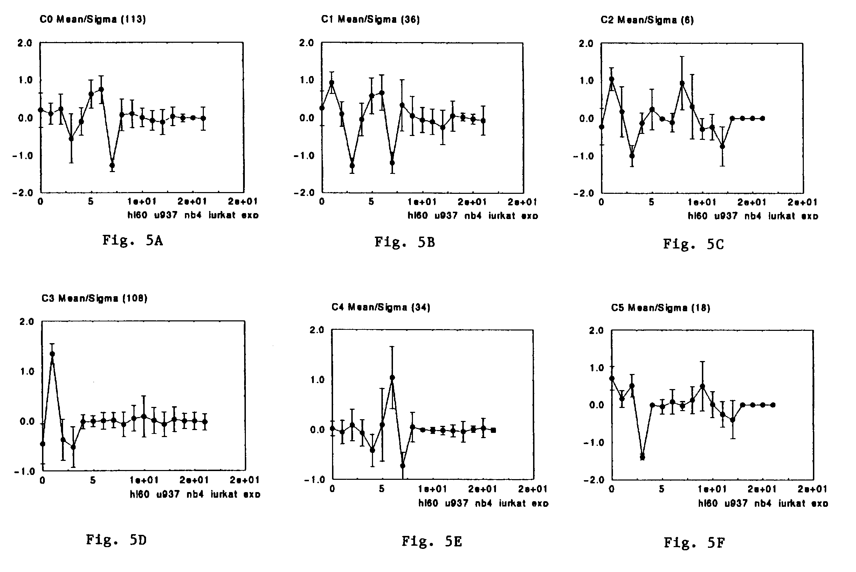

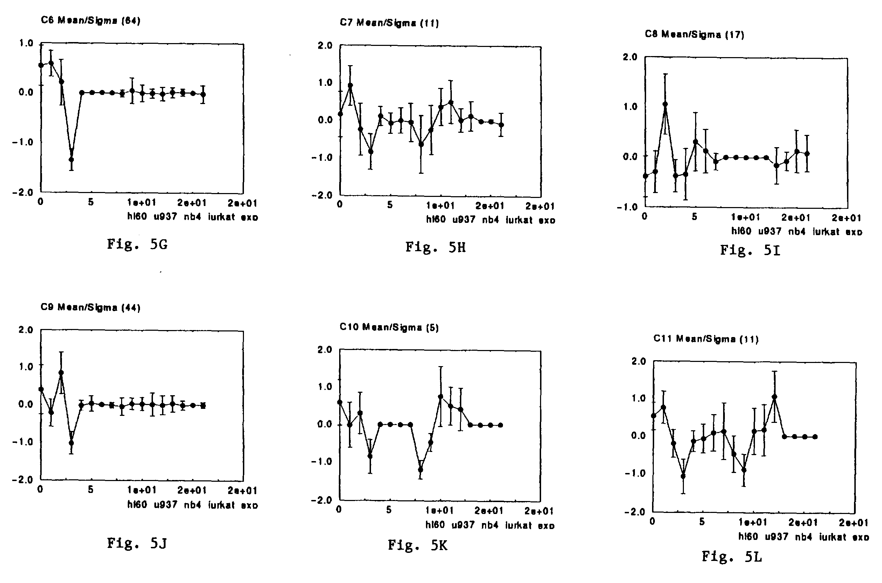

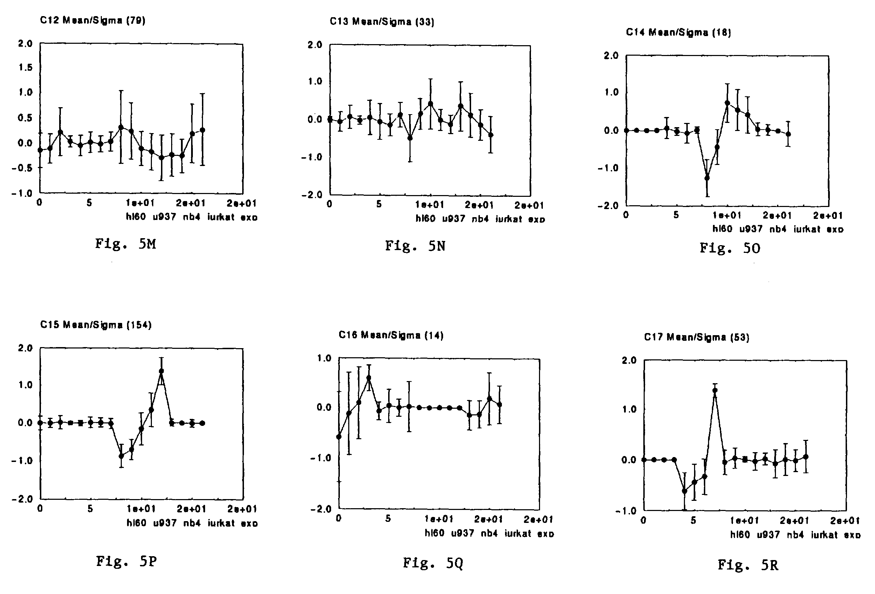

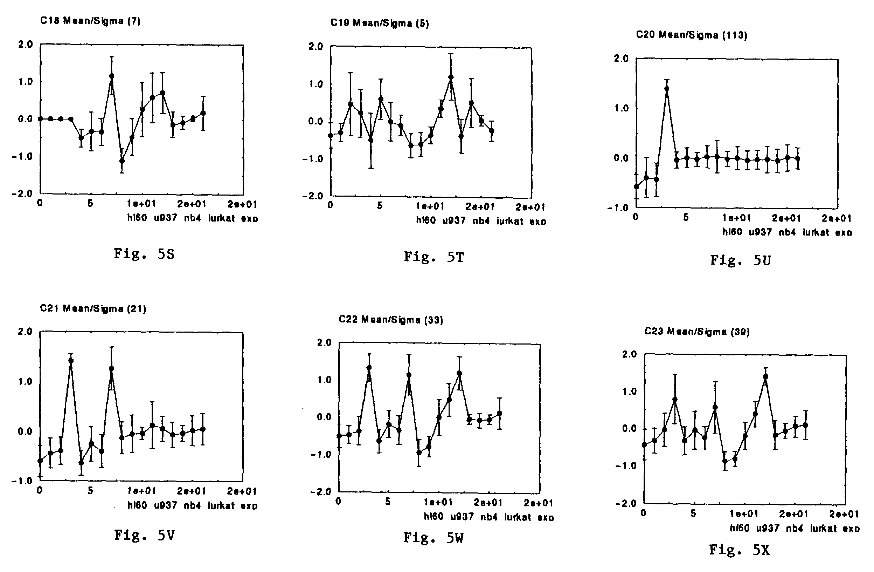

The present invention was applied to more complex datasets involving multiple cell lines: HL-60 and the similar myeloid cell line U937, which also undergoes macrophage differentiation in response to TPA; Jurkat, a T-cell line that acquires many hallmarks of T-cell activation in response to TPA; and NB4, an acute promyelocytic leukemia cell line that undergoes neutrophilic differentiation in response to all-trans retinoic acid (ATRA). A total of 17 RNA samples were generated, yielding 6416 datapoints in 17-dimensional space. Of these, 1036 genes passed the variation filter. The genes were classified with a 6x4 SOM (Figure 5A-X), thereby grouping the 1036 genes into 24 categories. See http://www.genome.wi.mit.edu/MPR for the entire database.

Cluster 21 contains 21 genes induced in the closely related cell lines HL-60 and U937, while the adjacent clusters 17 and 20 contain genes induced in one of the two lines. This indicates that while HL-60 and U937 have similar macrophage maturation responses to TPA stimulation, there are transcriptional responses tat distinguish the two cell lines. Cluster 22 contains genes upregulated in the three myeloid lines, but not the lymphoid cell line Jurkat.

Cluster 15 contains 154 genes induced by ATRA in NB4 cells but not regulated in the other three cell lines. NB4 cells harbor at translocation that fuses the PML and RARα genes, resulting in a fusion protein that blocks normal neutrophil differentiation. ATRA stimulation restores neutrophil differentiation. This response is the presumed basis of "differentiation therapy", which is part of standard treatment for individuals with acute promyelocytic leukemia, but the precise mechanism of differentiation remains uncertain.

Most of the genes in Cluster 15 encode markers of neutrophil differentiation (such as GCSF receptor, CD59 and Defensin α4) or proteins known to be induced by retinoic acid in various systems (such as the RIG-E gene and the interferon inducible genes IFI56, INP10 and IRF1). Some unexpected genes, however, provide novel and potentially interesting insights into NB4 differentiation.

Of the genes showing unexpected ATRA regulation, the most strongly induced was the G0S2 gene, which encodes a protein of unknown function reported as a cyclohexamide inducible protein in T-cells 24. Russell, L. & Forsdyke, D. (1991). *DNA Cell Biol* 10, 581-591. Northern analysis confirmed G0S2 induction as early as 6 hours following ATRA treatment of NB4 cells. The Northern Blot analysis of G0S2 Regulation was performed by subjecting RNA with a G0S2 probe. The blots were then reprobed for GAPDH as a loading control. Cells were treated wit the neutrophil differentiating agents all trans retinoic acid (RA) or DMSO for the times indicated in hours. NB4-S1 is an RA-sensitive subclone of NB4. NB4-R1 and NB4-R2 are subclones which fail to differentiate following RA treatment. NB4-R2 has a point mutation in PML/RARα; the mechanism of RA resistance in NB4-R1 is unknown. Interestingly, we also found that G0S2 is not upregulated in ATRA-induced neutrophildifferentiation of HL-60 cells (which lack PML/RARα ); in DMSO-induced neutrophildifferentiation of NB4 cells; or in ATRA-stimulation of ATRA-resistant NB4 cells (carrying an inactivating point mutation in the PML/RARα fusion). Whether G0S2 induction is seen in individuals treated with ATRA *in vivo* remains to be determined, but its early induction in NB4 cells is consistent with the hypothesis that G0S2 is a candidate PML/RARα-specific, ATRA-mediated regulator of neutrophil differentiation.

Another interesting observation is the specific induction in NB4 cells of two genes, LMP7 and UBE1L, related to ubiquitin-mediated proteolysis. Proteasome-dependent degradation of the leukemogenic PML/RARα fusion protein has been shown to occur following ATRA stimulation and is thought to be a critical step in differentiation therapy, but the mechanism has been previously unknown. Induction of LMP7, encoding a chain of the multi-subunit proteasome, is consistent with regulation of proteolysis though induction of specific proteasome subunits. In addition, LMP7 has been recently shown to be regulated by the wild type PML protein. UBE1L encodes a protein highly similar to the ubiquitin-activating enzyme E1, involved in ubiquitination of proteins targeted for degradation. The fact that UBE1L is specifically induced, while E1 itself is constitutively expressed in NB4 cells, raises the possibility that degradation of the PML/RAR$\alpha$ protein in response to ATRA is achieved through transcriptional induction of specific components of the proteolytic apparatus.

Example 4: Discussion of the Results for the Yeast Cell Cycle and Hematopoietic Differentiation Gene Expression Pattern.

Comparative expression studies have long been known to provide important insight into biological processes. Such studies have historically proceeded one gene at a time, but the advent of array technologies has now made it possible to collect data on thousands of genes simultaneously. Global views of gene expression reveal previously unrecognized patterns of gene regulation.

Several recent papers, such as the study by Chu, S., *et al., Science* 282, 699-705 (1998), have employed hierarchical clustering algorithms to organize genes into a phylogenetic tree, reflecting similarity in expression patterns. Hierarchical clustering of 6,000 genes results in 5,999 nested clusters. The interpretation of these clusters and the recognition of the fundamental patterns is subject to error because the interpretation is left to the observer.

SOMs take a fundamentally different approach. They attempt to provide an 'executive summary' of a massive dataset, by extracting then most prominent patterns (where n is the number of nodes in the geometry) and arranging them so that similar patterns occur as neighbors in the SOM. As with all exploratory data analysis tools, the use of SOMs involves inspection of the data to extract insights.

SOMs have many desirable mathematical properties, including scaling well to large datasets. SOMs have been proven to be valuable in analyses involving hundreds of experiments having gene expression data.

The examples presented herein illustrate the value of present invention which utilizes SOMs. Cell-cycle periodicity was automatically recovered as among the most prominent patterns during yeast growth. Analysis of more complex datasets of hematopoietic differentiation identified the genes and pathways previously known to be important in this process, and generated new hypotheses. The success of the SOM methodology in identifying the predominant gene expression patterns in these well-characterized model systems indicate that genome-wide expression profiling, together with appropriate computational tools, provides valuable insights into biological processes which have not previously been molecularly understood.

Example 5: Protocols Utilized in Expression Analysis

The following protocols were used in determining expression analysis of the yeast and macrophage differentiation.

First strand cDNA synthesis was performed as follows:

1. Add 10 uL total RNA (20 ug) ib DEPC H20 1uL 100 pmol/ul T7-(T)24 primer (GGCCAGTGAATTGTAATACGACTCACTATAGGGAGGCGG-(T)24)
2. Mix (quick spin if needed)
3. Heat @ 70C, 10 min
4. Put in ice bucket
5. Add on ice to RNA/primer mix:

- 4 ul 5X 1st Strand Buffer
- 2uL.1M DTT
- 1 ul 10mM dNTPs

6. Heat @ 37, 2min
7. Add 2 uL SSII RT (400 U total)
8. Mix (quick spin if needed)
9. Heat @ 42C, 1 hour

10. Proceed to "Second strand cDNA synthesis"

Second strand cDNA synthesis was performed as follows:

1. Ice all reagents and 1st strand tubes
2. Add to 1st strand tubes:

- 91.33 uL DEPC H20
- 30 uL 5X 2nd Strand Buffer
- 4 uL DNA POL I (40 Units)
- 3 uL 10 mM dNTPs
- 1 uL DNA Ligase (10 Units)
- .67 uL RNase H (2 Units)

3. Mix (quick spin if needed)
4. Incubate @ 16°C, 2 hours
5. Store@-80C

Clean-up of dscDNA was performed as follows:

1. Spin Phase-Lock tubes @ max, 30 sec
2. Add all of the cDNA reaction (approx. 150 uL)
3. Add equal volume buffer saturated phenol (or phenol/chloroform)
4. Vortex lightly
5. Spin @ max, 2 min
6. Transfer upper phase to new tube
7. Add

- 1/2X volume 7.5 M NH40Ac (75 uL)
- 2.5X volume 100% EtOH (375 uL)
- 1 uL Glycogen (20 mg/mL)

8. Mix
9. Spin @ max, R.T., 20 min
10. Decant supernatant (watch for pellet)
11. Wash pellet twice wit 80% EtOH
12. Speed vacuum to dry
13. Resuspend in 1.5 uL DEPC H2O

In Vitro Transcription (IVT) was performed as follows:

1. Thaw and room temperature all reagents
2. Make NTP mix (per tube):

- 2 uL 75 mM ATP
- 2 uL 75 mM GTP
- 1.5 uL 75 mM CTP
- 3.75 uL 10 mM Bio-11-CTP
- 3.75 uL 10 mM Bio-16-CTP
- 2 uL 10X Buffer

3. Add to cleaned dscDNA tube:

- 16.5 uL NTP mix
- 2 uL Enzyme mix (as provided in the kit)

4. Mix (quick spin if needed)
5. Incubate @ 37 C, 6 hours

IVT Clean-up was performed as follows:

1. Add to IVT reaction tube:

- 80 uL DEPC H20
- 350 uL RLT buffer

2. Mix
3. Add 250 uL 100% EtOH
4. Transfer sample to RNeasy spin column
5. Spin @ max, 15 sec
6. Transfer spin column to new collection tube
7. Add 500 uL RPE buffer
8. Spin @ max, 15 sec
9. Transfer spin column to new collection tube
10. Add 500 uL RPE buffer
11. Spin @ max, 2 min
12. Transfer spin column to new collection tube
13. Add 50 uL DEPC H2O to membrane of spin column
14. Let soak for 4 min
15. Spin @ max, 1 min
16. Repeat 13-15 using 1st elution as the 2nd elution
17. Take OD (1:50 dilution)
18. Run on a 1% agarose gel using denaturing sample buffer (See Appendix A)

Fragmentation of cRNA was performed as follows:

1. Add to separate tube:

- 40 ug cRNA (volume CANNOT exceed 64 uL)
- X uL 5X Fragmentation Buffer

Based on the volume of your cRNA, add the appropriate volume of 5X Fragmentation Buffer and adjust volume with DEPC H2O.
For example,
if you had 40 ug in 40 uL:
40 uL cRNA (40 ug)
10 uL 5X Fragmentation Buffer
50 uL Total Volume
or
40 ug in 50 uL:
50 uL cRNA (40 ug)
13 uL 5X Fragmentation Buffer
2 uL DEPC H2O
65 uL Total Volume

2. Mix
3. Heat @ 95, 35 min

4. Add:

- 450 uL 2X STT
- 9 uL 10 mg/mL Herring Sperm DNA
- 9 uL 948 Congrol Oligo or Control Oligo B2 (5'-Bio-GTCAAGATGCTACCGTTCA-3')
- 9 uL 100X Bio B, C, D, and Cre
- 0.5 mg/ml acetylated BSA

5. Adjust volume with DEPC H2O to 900 uL total volume

Gel using Denaturing Sample Buffer was prepared as follows:

1. Make Sample Buffer:

- .05 uL 10mg/mL Ethidium Bromide
- .5 uL 10X MOPS
- 5 uL deionized-Formamide
- 1.75 uL 37% Formaldehyde
- 1 uL 10X Loading Dye
- 1.7 uL DEPC H2O

2. Add 10 uL Sample Buffer to each sample and controls to be run
3. Heat @ 65 C, 10 min
4. Run on 1% Agarose gel

Example 6: Hematopoeitic Differentiation Across Four Cell Lines, HL60, U937, NB5 and Jurkat were Rescaled:

This dataset combines expression data from four different cell lines: HL-60 and U937, two myeloid cell lines which undergo macrophage differentiation in response to TPA; NB4, an acute promyelocytic leukemia cell line that undergoes neutrophilic differentiation in response to all-trans retinoic acid (ATRA), and Jurkat, a T-cell line that acquires many hallmarks of T-cell activation in response to TPA. The dataset contains a total of 17 columns:

4 time points for UL60 (0, 0.5, 4 and 24 hours),
4 time points for U937 (0, 0.5, 4 and 24 hours),
5 time points for NB4 (0, 5.5, 24, 48 and 72 hours),
4 time points for Jurkat (0, 0.5, 4 and 24 hours).

There are a total of 6416 rows (genes). This data was obtained using Affymetrix Hu6000 DNA micro-arrays.
The re-scaling factors used in this dataset are as follows:

| Time point: | Chip A | Chip B | Chip C | Chip D |
|---|---|---|---|---|
| HL60 t=0(baseline) | 1.0 | 1.0 | 1.0 | 1.0 |
| HL60 t=0.5 hours | 0.64 | 0.98 | 1.78 | 0.85 |
| HL60 t=4 hours | 0.81 | 0.86 | 1.87 | 0.93 |
| HL60 t=24 hours | 0.74 | 0.75 | 1.51 | 0.51 |
| U937 t=0 (baseline) | 1.0 | 1.0 | 1.0 | 1.0 |
| U937 t=0.5 hours | 1.35 | 2.21 | 1.12 | 1.58 |
| U937 t=4 hours | 1.28 | 2.83 | 0.87 | 1.45 |
| U937 t=24 hours | 1.01 | 0.99 | 0.49 | 0.76 |

(continued)

| Time point: | Chip A | Chip B | Chip C | Chip D |
|---|---|---|---|---|
| NB4 t=0 (baseline) | 1.0 | 1.0 | 1.0 | 1.0 |
| NB4 t=5.5 hours | 1.33 | 1.33 | 0.84 | 1.56 |
| NB4 t=24 hours | 1.31 | 1.30 | 1.20 | 2.72 |
| NB4 t=48 hours | 0.69 | 1.31 | 0.95 | 1.73 |
| NB4 t=72 hours | 1.17 | 1.02 | 0.98 | 1.57 |
| Jurkat t=0 (baseline) | 1.0 | 1.0 | 1.0 | 1.0 |
| Jurkat t=0.5 hours | 1.69 | 0.59 | 0.57 | 1.04 |
| Jurkat t=4 hours | 1.06 | 0.94 | 0.70 | 1.15 |
| Jurkat t=24 hours | 1.18 | 1.05 | 0.69 | 0.76 |

Example 7: HL60 Macrophage Differentiation Datasets were Rescaled:

This dataset contains four time points measurements corresponding to a differentiation time course of HL60 cells. These cells undergo macrophage differentiation upon treatment with the phorbol ester TPA. Nearly 100% of HL-60 cells become adherent and exit the cell cycle within 24 hours of TPA treatment. To monitor this process at the transcriptional level, cells were harvested at 0, 0.5, 4 and 24 hrs after TPA stimulation. PolyA+ RNA was isolated, double-stranded cDNA was prepared, and i*n vitro* transcription in the presence of biotinylated nucleotides was used to create labeled antisense cRNA. The samples were then hybridized to expression-monitoring arrays from Affymetrix, Inc., containing oligonucleotide probes for 5223 known human genes and 1193 expressed sequence tags (ESTs), and hybridization intensities were determined for each gene. This data was obtained using Affymetrix Hu6000 DNA micro-arrays.
The re-scaling factors used in this dataset are as follows:

| Time point: | Chip A | Chip B | Chip C | Chip D |
|---|---|---|---|---|
| t=0 (baseline) | 1.0 | 1.0 | 1.0 | 1.0 |
| t=0.5 hours | 0.64 | 0.98 | 1.78 | 0.85 |
| t=4 hours | 0.81 | 0.86 | 1.87 | 0.93 |
| t=24 hours | 0.74 | 0.75 | 1.51 | 0.51 |

While this invention has been particularly shown and described with references to preferred embodiments thereof, it will be understood by those skilled in the art that various changes in form and details may be made therein without departing from the spirit and scope of the invention as defined by the appended claims.

**Claims**

1. In a computer system, a method for clustering a plurality of datapoints, wherein each datapoint is a series of gene expression values, wherein the method comprises:

   a) receiving the gene expression values of the datapoints;
   b) using a self organizing map, clustering the datapoints such that the datapoints that exhibit similar patterns are clustered together into respective clusters; and
   c) providing an output indicating the clusters of the datapoints.

2. The method of Claim 1, further comprising filtering out any datapoints that exhibit an insignificant change in the gene expression value, such that working datapoints remain, and for example further comprising normalizing the gene expression value of the working datapoints.

3. In a computer system, a method for grouping a plurality of datapoints, wherein each datapoint is a series of gene expression values, wherein the method comprises:

    a) receiving gene expression values of the datapoints;
    b) filtering out any datapoints that exhibit an insignificant change in the gene expression value, such that working datapoints remain;
    c) normalizing the gene expression value of the working datapoints;
    d) using a self organizing map, grouping the working datapoints such that the datapoints that exhibit similar patterns are grouped together into respective clusters; and
    e) providing an output indicating the groups of the datapoints.

4. The method of any one of Claims 1, 2 and 3, wherein the gene expression values are obtained from a gene that is subjected to at least one condition.

5. The method of Claim 4, wherein the step of receiving includes receiving gene expression values of datasets, wherein a dataset is a series of gene expression values across multiple genes for a condition.

6. The method of any one of the preceding claims, wherein the self organizing map is formed of a plurality of Nodes, N, and clusters or groups the datapoints according to a competitive learning routine, which for example is:

$$f_{i+1}(N) = f_i(N) + \tau(d(N,N_p), i) (P - f_i(N))$$

wherein i = number of iterations, N = the node of the self organizing map, $\tau$ = learning rate, P = the subject working datapoint, d = distance, $N_p$ = node that is mapped nearest to P, and $f_i(N)$ is the position of N at i.

7. The method of any one of the preceding claims, wherein the step of providing includes displaying at least one representative datapoint from each cluster or group.

8. The method of Claim 2 or Claim 5, wherein the step of normalizing the gene expression value comprises determining the ratio of a) difference between the subject gene expression value and the average gene expression value across datasets, and b) the standard deviation of the gene expression value across datasets.

9. The method of any one of the preceding claims further comprising rescaling the gene expression values to account for variations across multiple conditions.

10. A computer apparatus for clustering a plurality of datapoints, wherein each datapoint is a series of gene expression values, wherein the apparatus comprises:

    a) a source of gene expression values of the datapoints;
    b) a processor routine coupled to receive datapoints from the source, the processor routine utilizing a self organizing map for clustering datapoints such that the datapoints that exhibit similar patterns are clustered together into respective clusters; and
    c) an output device, coupled to the processor routine, for indicating the clusters of the datapoints.

11. The computer apparatus of Claim 10, further comprising a filter, coupled to the source, for filtering out any of the datapoints that exhibit an insignificant change in the gene expression value, such that working datapoints remain, and for example further comprising a normalizing processor coupled to the filter, for normalizing the gene expression value of the working datapoints.

12. A computer apparatus for grouping a plurality of datapoints, wherein each datapoint is a series of gene expression values, wherein the apparatus comprises:

    a) a source of gene expression values of the datapoints;
    b) a filter, coupled to the source, for receiving the gene expression values and filtering out any of the datapoints that exhibit an insignificant change in the gene expression value, such that working datapoints remain;
    c) a normalizing process, coupled to the filter, for normalizing the gene expression value of the working datapoints;
    d) a processor routine that is responsive to the normalizing process and utilizes a self organizing map for grouping the working datapoints such that the datapoints that exhibit similar patterns are grouped together into respective groups; and

e) an output device, coupled to the processor routine, for indicating the groups of the datapoints.

13. The apparatus of any one of Claims 10, 11 and 12, wherein the gene expression values are obtained from a gene that is subjected to at least one condition.

14. The apparatus of Claim 13, wherein the source further provides datasets, each dataset being a series of gene expression values across multiple genes for a condition.

15. The apparatus of Claim 11 or Claim 12, wherein the normalizing process of the gene expression value is determined according to the ratio of a) difference between the subject gene expression value and the average gene expression value across datasets, and b) the standard deviation of the gene expression value across datasets.

16. The apparatus of any one of Claims 10 to 15, wherein the self organizing map is formed of a plurality of Nodes, N, and clusters or groups the datapoints according to a competitive learning routine, which for example is:

$$f_{i+1}(N) = f_i(N) + \tau(d(N, N_p), i) (P - f_i(N))$$

wherein i = number of iterations, N = the node of the self organizing map, $\tau$ = learning rate, P = the subject working datapoint, d = distance, $N_p$ = node that is mapped nearest to P, and $f_i(N)$ is the position of N at i.

17. The apparatus of any one of Claims 10 to 16, wherein the output device comprises a display of at least one representative datapoint from each cluster or group.

18. A method for assessing expression patterns of two or more genes in cells, wherein the expression patterns are represented by a plurality of datapoints, wherein each datapoint is a series of gene expression values, wherein the method comprises:

a) receiving the gene expression values of the datapoints;
b) using a self organizing map, clustering the datapoints such that the datapoints that exhibit similar patterns are clustered together into respective clusters;
c) providing an output indicating the clusters of the datapoints; and
d) analyzing the output to determine the similarities or differences between the expression patterns of the genes.

19. The method of Claim 18, further comprising rescaling the gene expression values to account for variations across multiple conditions.

20. The method of Claim 18 or Claim 19, wherein the self organizing map is formed of a plurality of Nodes, N, and clusters the datapoints according to a competitive learning routine.

21. A method of determining relatedness of expression patterns of two or more genes, wherein the expression patterns are represented by a plurality of datapoints, wherein each datapoint is a series of gene expression values, wherein the method comprises:

a) receiving the gene expression values of the datapoints;
b) using a self organizing map, clustering the datapoints such that the datapoints that exhibit similar patterns are clustered together into respective clusters;
c) providing an output indicating the clusters of the datapoints; and
d) analyzing the output to determine the similarities and/or differences between the expression patterns of the genes, thereby determining the relatedness of two or more genes.

22. The method of any one of Claims 18 to 21, wherein the gene expression values are obtained from a gene that is subjected to at least one condition, and for example wherein a dataset is a series of gene expression values across multiple genes for a condition.

23. The method of Claim 22, further comprising filtering out any datapoints that exhibit an insignificant change in the gene expression value, such that working datapoints remain, and for example further comprising normalizing the gene expression value of the working datapoints.

24. A method for characterizing expression patterns of a plurality of genes of a sample having unknown characteristics, wherein the sample from an individual is obtained and subjected to a multiplicity of diagnostic tests, and the expression patterns of the genes for the diagnostic tests are represented by a plurality of datapoints, wherein the datapoint is a series of gene expression values across multiple genes for the diagnostic test, wherein the method comprises:

a) receiving the gene expression values of the datapoints from the diagnostic tests;
b) using a self organizing map, clustering the datapoints such that the datapoints that exhibit similar patterns are clustered together into respective clusters;
c) providing an output indicating the clusters of the datapoints; and
d) comparing the output of the gene expression patterns of the unknown sample against a control, thereby characterizing gene expression patterns of the sample.

25. The method of Claim 24, wherein the gene expression values across multiple genes for the diagnostic test is obtained from a gene subjected to at least one condition, and for example wherein a dataset is a series of gene expression values from a gene subjected to the diagnostic tests.

26. The method of Claim 25, wherein the sample from the individual is selected from cells, lysed cells, cellular material suitable for determining gene expression, and material containing gene expression products.

27. The method of any one of Claims 18 to 26 further comprising normalizing the gene expression value of the datapoints, and for example wherein the self organizing map is formed of a plurality of Nodes, N, and clusters the datapoints according to a competitive learning routine.

28. The method of any one of Claims 18 to 27 wherein the self organising map clusters the datapoints according to a competitive learning routine which is:

$$f_{i+1}(N) = f_i(N) + \tau(d(N,N_p), i) (P - f_i(N))$$

wherein i = number of iterations, N = the node of the self organizing map, $\tau$ = learning rate, P = the subject working datapoint, d = distance, $N_p$ = node that is mapped nearest to P, and $f_i(N)$ is the position of N at i; and for example wherein the step of normalizing the gene expression value comprises determining the ratio of a) difference between the subject gene expression value and the average gene expression value across datasets, and b) the standard deviation of the gene expression value across datasets.

29. A method of identifying a drug target from the expression patterns of two or more genes from cells, the expression patterns are represented by a plurality of datapoints, and wherein each datapoint is a series of gene expression values, wherein the method comprises:

a) obtaining cells that express genes,
b) subjecting the cells to an agent or condition for testing the drug target,
c) measuring gene expression from the cells subjected to the agent or condition, and from a control, to obtain the gene expression values,
d) receiving the gene expression values of the datapoints;
e) using a self organizing map, clustering the datapoints such that the datapoints that exhibit similar patterns are clustered together into respective clusters;
f) comparing the clusters from the genes that have been subjected to the agents or condition with a control; and
g) providing an output indicating clusters, to thereby determine the drug target.

30. The method of Claim 29, further comprising filtering out any datapoints that exhibit an insignificant change in the gene expression value, such that working datapoints remain, and for example further comprising normalizing the gene expression value of the working datapoints.

31. The method of Claim 30, wherein the self organizing map clusters the datapoints according to:

$$f_{i+1}(N) = f_i(N) + \tau(d(N,N_p), i) (P - f_i(N))$$

wherein i = number of iterations, N = the node of the self organizing map, $\tau$ = learning rate, P = the subject working datapoint, d = distance, $N_p$ = node that is mapped nearest to P, and $f_i(N)$ is the position of N at i.

32. A drug target identified or identifiable by the method of any one of Claims 29 to 31.

33. A computer readable product having a program recorded thereon loadable into the internal memory of a digital computer and comprising software code portions for performing the steps of the method of any one of claims 1 to 9; or 18 to 31.

Fig. 1

Input Source 17

I/O Process
19

13

11

15

Storage
21

I/O Process
23

Output

Fig. 2

C0 Mean/Sigma (44)

mitosis exo norm9.dat

Fig. 3A

C1 Mean/Sigma (32)

mitosis exo norm9.dat

Fig. 3B

C2 Mean/Sigma (28)

mitosis exo norm9.dat

Fig. 3C

C3 Mean/Sigma (17)

mitosis exo norm9.dat

Fig. 3D

C4 Mean/Sigma (69)

mitosis exo norm9.dat

Fig. 3E

C5 Mean/Sigma (23)

mitosis exo norm9.dat

Fig. 3F

C6 Mean/Sigma (15)

Fig. 3G

C7 Mean/Sigma (24)

Fig. 3H

C8 Mean/Sigma (16)

Fig. 3I

C9 Mean/Sigma (16)

Fig. 3J

C10 Mean/Sigma (28)

Fig. 3K

C11 Mean/Sigma (22)

Fig. 3L

Fig. 3O

Fig. 3R

Fig. 3N

Fig. 3Q

Fig. 3M

Fig. 3P

Fig. 3S

Fig. 3T

Fig. 3U

Fig. 3V

Fig. 3W

Fig. 3X

Fig. 3A1

Fig. 3Z

Fig. 3Y

Fig. 3D1

Fig. 3C1

Fig. 3B1

Fig. 3E1

Fig. 3F1

Fig. 3G1

Fig. 4A

Fig. 4B

Fig. 4C

Fig. 4D

Fig. 4E

Fig. 4F

32

C6 Mean/Sigma (48)

C7 Mean/Sigma (18)

C8 Mean/Sigma (2)

Fig. 4G

Fig. 4H

Fig. 4I

C9 Mean/Sigma (40)

C10 Mean/Sigma (142)

C11 Mean/Sigma (32)

Fig. 4J

Fig. 4K

Fig. 4L

C0 Mean/Sigma (113)

C1 Mean/Sigma (36)

C2 Mean/Sigma (6)

hl60 u937 nb4 iurkat exo

hl60 u937 nb4 iurkat exo

hl60 u937 nb4 iurkat exo

Fig. 5A

Fig. 5B

Fig. 5C

C3 Mean/Sigma (108)

C4 Mean/Sigma (34)

C5 Mean/Sigma (18)

hl60 u937 nb4 iurkat exo

hl60 u937 nb4 iurkat exo

hl60 u937 nb4 iurkat exo

Fig. 5D

Fig. 5E

Fig. 5F

Fig. 5G

Fig. 5H

Fig. 5I

Fig. 5J

Fig. 5K

Fig. 5L

Fig. 5O

Fig. 5N

Fig. 5M

Fig. 5R

Fig. 5Q

Fig. 5P

Fig. 5S

Fig. 5T

Fig. 5U

Fig. 5V

Fig. 5W

Fig. 5X

# Yeast Cell Cycle Analysis Project

| Ch-1 (Green) | Ch-2 (Red) | Data File | Image File |
|---|---|---|---|
| Cln/Clb Experiments | | | |
| Time Zero | Clb2 expt2 | Data | Image |
| Time Zero | Cln3 expt2 | Data | Image |
| Time Zero | Clb2 expt1 | Data | Image |
| Time Zero | Cln3 expt1 | Data | Image |
| - Galactose | - Galactose | Data | Image |
| Pheromone Experiments | | | |
| asynchronous | 000 min | Data | Image |
| asynchronous | 007 min | Data | Image |
| asynchronous | 014 min | Data | Image |
| asynchronous | 021 min | Data | Image |
| asynchronous | 028 min | Data | Image |
| asynchronous | 035 min | Data | Image |
| asynchronous | 042 min | Data | Image |
| asynchronous | 049 min | Data | Image |
| asynchronous | 056 min | Data | Image |
| asynchronous | 063 min | Data | Image |
| asynchronous | 070 min | Data | Image |
| asynchronous | 077 min | Data | Image |
| asynchronous | 084 min | Data | Image |
| asynchronous | 091 min | Data | Image |
| asynchronous | 098 min | Data | Image |
| asynchronous | 105 min | Data | Image |
| asynchronous | 112 min | Data | Image |
| asynchronous | 119 min | Data | Image |
| cdc15 Experiments | | | |
| asynchronous | 010 min | Data | Image |
| asynchronous | 030 min | Data | Image |
| asynchronous | 050 min | Data | Image |
| asynchronous | 070 min | Data | Image |
| asynchronous | 080 min | Data | Image |
| asynchronous | 090 min | Data | Image |
| asynchronous | 100 min | Data | Image |
| asynchronous | 110 min | Data | Image |
| asynchronous | 120 min | Data | Image |

Figure 6A

| asynchronous | 120 min | Data | Image |
|---|---|---|---|
| asynchronous | 130 min | Data | Image |
| asynchronous | 140 min | Data | Image |
| asynchronous | 150 min | Data | Image |
| asynchronous | 160 min | Data | Image |
| asynchronous | 160 min | Data | Image |
| asynchronous | 170 min | Data | Image |
| asynchronous | 180 min | Data | Image |
| asynchronous | 190 min | Data | Image |
| asynchronous | 200 min | Data | Image |
| asynchronous | 210 min | Data | Image |
| asynchronous | 220 min | Data | Image |
| asynchronous | 240 min | Data | Image |
| asynchronous | 250 min | Data | Image |
| asynchronous | 270 min | Data | Image |
| asynchronous | 290 min | Data | Image |
| **Elutriation Experiments** | | | |
| Ref Pool | 0 min | Data | Image |
| Ref Pool | 30 min | Data | Image |
| Ref Pool | 60 min | Data | Image |
| Ref Pool | 90 min | Data | Image |
| Ref Pool | 120 min | Data | Image |
| Ref Pool | 150 min | Data | Image |
| Ref Pool | 180 min | Data | Image |
| Ref Pool | 210 min | Data | Image |
| Ref Pool | 240 min | Data | Image |
| Ref Pool | 270 min | Data | Image |
| Ref Pool | 300 min | Data | Image |
| Ref Pool | 330 min | Data | Image |
| Ref Pool | 360 min | Data | Image |
| Ref Pool | 390 min | Data | Image |

Figure 6B